# FASCICULE DE BREVET EUROPEEN

(11) **EP 3 043 783 B1**
(45) Date de publication et mention de la délivrance du brevet: **13.11.2019**
(21) Numéro de dépôt: 14762037.1
(22) Date de dépôt: 12.09.2014
(51) Int. Cl.: A61K 31/047, A61K 31/723, A61K 31/79, A61K 45/06

(54) **COMPLEXE LUBRIFIANT POUR LA BOUCHE**
MUNDSPEICHELERSATZSTOFFE
LUBRICATING COMPLEX FOR THE MOUTH

(30) Priorité: 13.09.2013 FR 1358841
(43) Date de publication de la demande: 20.07.2016
(73) Titulaire: Pierre Fabre Médicament, 92100 Boulogne-Billancourt (FR)
(72) Inventeur: BAUDONNET, Lara, F-31600 Eaunes (FR); BOUYRIE, Julie, F-31270 Cugnaux (FR); CORDOLIANI, Jean-François, F-31570 Sainte Foy D'aigrefeuille (FR); OBE, Hélène, F-31270 Villeneuve Tolosane (FR); TRANNOY, Philippe, F-31400 Toulouse (FR)
(74) Mandataire: Regimbeau
(86) Numéro de dépôt international: PCT/EP2014/069541
(87) Numéro de publication internationale: WO 2015/036564

(56) Documents cités:
- WO-A1-2006/013081
- Anonymous: "GNPD - Moisturizing Mouthwash & Mouth Spray", , octobre 2010 (2010-10), XP055109102, Extrait de l'Internet: URL:http://www.gnpd.com/sinatra/recordpage /1413244/from_search/ZN4ioqIvab/?__cc=1 [extrait le 2014-03-20]
- SHIRODARIA SOHA ET AL: "Subjective assessment of a new moisturizing mouth spray for the symptomatic relief of dry mouth.", THE JOURNAL OF CLINICAL DENTISTRY 2006, vol. 17, no. 2, 2006, pages 45-51, XP009177088, ISSN: 0895-8831
- Anonymous: "Biotène Mouth Spray - Mouthsprays for Dry Mouth - Dry Mouth Relief | Biotène", , 2013, XP002722047, Extrait de l'Internet: URL:http://www.biotene.com/dry-mouth-produ cts/mouth-spray [extrait le 2014-03-20]
- Balas Kim: "Marshmallow, health benefits of marshmallow", , 2008, XP055109152, Extrait de l'Internet: URL:http://thehealthytruth.net/marshmallow .html [extrait le 2014-03-20]

## Description

La présente invention a pour objet des compositions pharmaceutiques comprenant au moins un agent humectant, un agent gélifiant et un agent filmogène pour son utilisation dans le traitement symptomatique de l'hyposialie ou de l'asialie.

La salive est un produit très complexe présentant de nombreuses fonctions : nettoyage, tampon, lubrification, perception du goût, protection contre la déminéralisation, défense. La salive normale contient des anticorps, des enzymes et des solutions tampon. Elle joue un rôle important dans la parole, le goût, la mastication, la déglutition, la digestion, la nutrition ainsi que dans la protection de la bouche, des lèvres et de l'œsophage. Ainsi, la salive exerce un rôle clé dans le maintien de la santé orale en raison de plusieurs facteurs. Premièrement le flux salivaire exerce une action de nettoyage sur les surfaces bucco dentaires. Ensuite, la salive favorise la reminéralisation des lésions carieuses débutantes *via* son effet tampon et la présence d'ions minéraux. De plus la présence d'enzyme telle que l'amylase est essentielle pour la digestion, processus long et complexe débutant dans la bouche. Pour terminer, la présence de substances antibactériennes permet une inhibition du métabolisme des micro-organismes pathogènes bucco dentaires. Ces raisons font que la salive joue un rôle essentiel dans le processus de régulation qui vise à contrebalancer l'action pathogène des bactéries de la flore buccale, et cette dernière ne peut jouer son rôle protecteur que lorsqu'elle est produite en quantité suffisante par les glandes salivaires. Mais la salive joue également un rôle de lubrifiant puisqu'elle permet un meilleur glissement des muqueuses et des dents entre elles et donne aussi une sensation d'humectation de la bouche, essentielle à la parole, la déglutition, la mastication et finalement au bien être. La salivation est un réflexe inné, mais qui peut être acquis, comme l'a montré l'expérience de Pavlov. La sécrétion de salive est très variable d'un individu à l'autre pouvant aller de 500 ml à 1200 ml par jour, 70% d'origine parotidienne, 20% d'origine sous-maxillaire, 10% d'origine sublinguale et d'autres glandes accessoires qui sont disséminées sous toute la surface de la muqueuse buccale, labiales, jugales, palatines et vélaires. La sécrétion de repos serait d'environ 100 ml par jour alors que la sécrétion stimulée serait environ dix fois supérieure. La production de salive après chaque repas est essentielle car la salive est le seul système de défense naturel de l'organisme contre les pathogènes bucco-dentaires. De nombreuses études montrent le rôle important de la salive comme facteur impliqué dans le développement des caries et des maladies parodontales.

Les défauts de la fonction salivaire dans le sens d'une diminution de sécrétion salivaire sont l'hyposialie et l'asialie. L'hyposialie est une diminution de la fabrication de salive par les glandes salivaires, qui provoque une sécheresse buccale. L'asialie est une absence totale de sécrétion de salive qui provoque une sécheresse totale. La xérostomie définit un état de sécheresse de la cavité buccale et des lèvres ressenti de façon subjective par le patient, traduisant une atteinte directe ou indirecte des glandes salivaires et se manifestant soit par une diminution du flux salivaire ou hyposialie soit par une sécrétion salivaire nulle ou asialie. La xérostomie est donc un symptôme.

L'hyposialie présente une prévalence importante environ 10% de la population générale et notamment chez les personnes âgées, 30% des personnes ayant plus de 65 ans en seraient atteintes. Elle entraîne des douleurs, des troubles fonctionnels (déglutition, mastication, phonation), des perturbations de l'appétit (dysphagie, dysgueusie, dénutrition avec complications chez les personnes âgées), des difficultés pour parler, des infections orales (caries à progression rapide, candidoses, ulcérations des muqueuses) responsables d'extractions dentaires multiples et d'intolérances aux prothèses, ainsi qu'une augmentation du risque de fausses routes, de pneumonies d'inhalation et d'infections nosocomiales pulmonaires, notamment lorsque les personnes âgées sont hospitalisées. L'absence ou la diminution de salive contribue au reflux œsophagien ainsi que classiquement à la sensation permanente de soif et à une modification de l'haleine. Il peut être difficile d'avaler un médicament avec moins de salive, cela peut entraîner la non-observance du traitement médicamenteux. La sécheresse de la bouche peut également retarder la dissolution des comprimés sublinguaux de nitroglycérine par exemple. La bouche sèche et ses conséquences pour des raisons tant physiologiques qu'esthétiques retentissent profondément sur l'humeur et le comportement social. A terme, des conséquences sur la nutrition entraînant une perte de poids et les plaisirs de l'oralité sont à craindre. Tous ces problèmes supplémentaires peuvent avoir un impact négatif sur la qualité de vie du patient.

Trois causes majeures et bien définies sont à l'origine d'une hyposialie, l'étiologie la plus courante est l'iatrogénie médicamenteuse. Plus de 500 médicaments, incluant 42 groupes pharmacologiques différents pourraient être à l'origine d'une hyposialie. Les médicaments qui induisent une xérostomie la plus intense sont ceux qui ont un impact direct sur le système nerveux autonome qui régule les sécrétions des glandes, comme les agents anticholinergiques, les bloqueurs adrénergiques, ceux qui ont un impact indirect sur le système nerveux central comme les agents antipsychotiques, les agents anxiolytiques mais aussi les médicaments qui augmentent l'excrétion des liquides comme les diurétiques. D'autres catégories comme les antihistaminiques, les anti-arythmiques, les antitussifs sont également régulièrement cités comme responsables d'une sécheresse buccale exacerbée. En cas d'hyposialie induite par les médicaments, il importe de rappeler que cette sécheresse peut concerner toutes les autres muqueuses dont celles de l'appareil génital. L'hyposialie survient également chez les personnes traitées par radiothérapie lors de cancers ORL (cancers de la bouche, de la langue, des amygdales, du larynx, du pharynx, des fosses nasales...). Les rayons détruisent toutes les glandes salivaires placées sur leur trajectoire. La troisième cause d'hyposialie est une maladie auto-immune, appelée « syndrome de Gougerot-Sjögren ». Cette maladie est caractérisée par une atteinte des glandes exocrines, en particulier des glandes salivaires et lacrymales, elle touche 1 à 3% de la population et plus souvent les femmes que les hommes (9/1), souvent après la ménopause. Ce syndrome peut être associé à une autre maladie auto-immune, le lupus érythémateux disséminé, la polyarthrite rhumatoïde... Enfin d'autres causes peuvent engendrer une hyposialie, une déshydratation même minime de l'organisme, lorsque celui-ci perd seulement 8% de son eau elle induit une inhibition de la sécrétion salivaire. Ainsi, la fièvre, la sudation excessive, le vomissement, la diarrhée, les pertes sanguines ou les brûlures qui causent une déshydratation peuvent donc aussi entraîner la sécheresse de la bouche. Une hyposialie peut également apparaître lors d'utilisation de drogues ou la consommation de tabac. Une lésion d'un nerf dans la région du cou et de la tête par une blessure ou une intervention chirurgicale peut également entraîner la sécheresse de la bouche. Une réduction de la capacité de mastication peut être à l'origine de l'établissement d'un régime alimentaire liquide ou d'aliments mous, ce qui provoque une diminution de l'écoulement de salive. Par ailleurs, des facteurs psychologiques comme le stress, l'anxiété ou des conditions dépressives sont également reliées à une xérostomie. Cependant, l'hyposalivation est normalement associée à la xérostomie induite par les médicaments alors que cette association n'est pas fréquente dans le cas d'une xérostomie reliée à des conditions psychologiques.

Toutes les hyposialies doivent être corrigées pour éviter tout risque d'apparition de pathologies bucco-dentaires, d'autant plus que le mode de vie moderne ne permet plus d'effectuer un brossage dentaire après chaque repas et tout particulièrement celui du déjeuner. L'absence de brossage dentaire associé à une déficience salivaire entraînera inéluctablement l'apparition de pathologies bucco-dentaires et il est donc essentiel de faire face à ce danger en stimulant la sécrétion salivaire après chaque repas.

Du fait que des possibilités de traitement causal de la xérostomie font toujours défaut jusqu'à présent, l'objectif thérapeutique se limite au seul soulagement symptomatique de la sécheresse buccale. Une telle amélioration peut être obtenue par différents moyens thérapeutiques, soit d'une part par des solutions pour des rinçages ou bains buccaux et d'autre part par des succédanés salivaires ou substitut salivaire ou encore salive artificielle, soit par une stimulation systémique des glandes salivaires.

En cas d'hyposialie iatrogène, il est possible d'arrêter le médicament ou le traitement responsable de la xérostomie mais cela semble difficilement concevable lorsque la plupart sont prescrits sur de longues périodes et concernent des pathologies graves. Pour le choix des stimulants salivaires, en raison du fait que la mastication stimule le flux salivaire, il est judicieux de recommander aux patients de consommer de préférence des aliments de consistance ferme. Vient ensuite la possibilité de boire beaucoup d'eau afin de maintenir la bouche hydratée, de boire des boissons gazeuses permettant d'humecter la muqueuse buccale et favorisant également parfois la sécrétion de salive, d'ajouter un système d'humidification dans la chambre à coucher pour augmenter l'humidité dans l'air, de respirer par le nez au lieu de la bouche autant que possible ou encore de sucer un bonbon acidulé, ou mastiquer une gomme mais sans sucre pour éviter les caries dentaires. Une autre possibilité de stimulation du flux salivaire consiste en l'administration de médicaments stimulateurs de la salive à effet systémique ou sialogogues, tels que la pilocarpine, le nicotinamide. Parmi les sialogogues systémiques, la pilocarpine s'est avérée la substance la plus efficace, elle est admise dans la plupart des pays à titre de médicament pour le traitement de l'hyposialie. La pilocarpine est un alcaloïde, extrait des feuilles de pilocarpus de différentes variétés jaborandi, pennatifolius, microphyllus.

La pilocarpine, par son action parasympathomimétique stimule les sécrétions des glandes exocrines, elle permet donc d'obtenir en général une élévation marquée du taux de flux salivaire et par conséquence une atténuation des symptômes de la sécheresse buccale. Toutefois, de nombreux patients présentant une xérostomie souffrent des symptômes de la sécheresse buccale en particulier durant la nuit, de ce fait les substances stimulatrices du flux salivaire administrées pendant la journée ne sont pas à même de les soulager à ces moments là. Mais ces substances ne permettent d'obtenir l'effet escompté que chez les patients possédants encore une certaine activité résiduelle des glandes salivaires. Il faut ajouter à cela que l'administration de médicaments à base de pilocarpine peut entraîner des effets secondaires indésirables importants tels que des nausées, des hypersudations, des sensations vertigineuses, des bouffées de chaleur ou des asthénies. La demande WO2005/067924 divulgue une composition pour le traitement des hyposialies à base de pilocarpine associée à au moins un polymère bio-adhésif de façon à permettre une dissolution et une fixation locale au niveau des tissus de la sphère bucco-pharyngée.

Aussi variées soient elles, toutes ces méthodes ne font que compenser le manque de salive et ne rétablissent pas durablement la fonction salivaire, ce qui à terme est l'objectif pour le patient.

Jusqu'à l'avènement de produits plus complexes de substitution salivaire, la littérature préconisait l'utilisation de bains de bouche non irritants, de solutions à base de bicarbonate de soude, de sérum physiologique ou de chlorhexidine pour atténuer les symptômes chez les patients souffrant d'hyposialie. De même, des solutions pour des rinçages buccaux, tels que le thé, les eaux minérales fluorées et le lait ont été préconisées en raison de leur efficacité de protection contre la carie dentaire. Il convient de recommander aux patients d'éviter la consommation de toutes les substances irritantes ou les aliments trop épicés, de même que toutes celles et ceux qui contiennent de l'alcool. Pour des raisons de protection des tissus dentaires durs, il ne faudrait en aucun cas utiliser des solutions pour des rinçages buccaux à pH acide et contenant un taux relativement élevé d'acides au titrage chez les patients possédants encore des dents naturelles, du fait d'un effet potentiellement délétère sur la dentine. Le recours aux solutions pour des bains de bouche ou des rinçages buccaux, voire à l'eau, le lait ou le thé, pour le soulagement des symptômes de la xérostomie est cependant grevé d'un inconvénient majeur. En effet, les propriétés physico-chimiques défavorables de ces solutions fait qu'il est nécessaire de répéter très souvent les applications pour assurer un effet mouillant suffisant et durable.

Le premier substitut salivaire a été mis au point il y a plusieurs décennies, l'ingrédient de base de ce succédané salivaire était la carboxyméthylcellulose et contenait en outre du calcium et du phosphate. Par la suite les fabricants ont ajouté du sorbitol aux salives artificielles afin de les rendre plus agréables au goût et d'en augmenter l'effet mouillant par la modification de la tension de surface, mais la combinaison de ces deux substances induit une augmentation de la viscosité des préparations par rapport à la salive naturelle. D'autres préparations de substitution de la salive contiennent en tant que substance active de base de la carboxyméthylcellulose de sodium, de la carboxyéthylcellulose, de l'hydroxyéthylcellulose, des mucines d'origine animale, de l'huile de lin, du sorbitol ou du polyéthylène-oxyde. Les succédanés salivaires se distinguent notamment par l'adjonction de différents composants inorganiques, la présence de certaines enzymes et par leur valeur de pH.

On peut notamment citer comme substitut salivaire, Novasial® à base de blanc d'œuf choisi pour ses caractéristiques rhéologiques et physiologiques proches de celles de la salive, de la gomme xanthane, du bicarbonate et du sorbitol. Biotène® est un dispositif médical à base de glycérol, de polymères et de gomme xanthane.

Idéalement, un substitut salivaire devrait être capable d'exercer un effet lubrifiant et mouillant sur l'ensemble des muqueuses et des tissus dentaires durs pendant une durée prolongée, il devrait protéger les muqueuses contre le dessèchement, tout en n'exerçant bien entendu aucun effet délétère sur l'organisme en général.

WO 2006/013081 décrit une composition pour traiter la xérostomie comprenant de la polyvinylpyrrolidone ou de la copolyvidone.

Ainsi, s'il existe un certain nombre de produits succédanés de la salive destinés au traitement symptomatique en cas de xérostomie, il reste toujours un réel besoin pour de nouvelles compositions faciles à utiliser avec une réelle efficacité et durant un laps de temps prolongé et surtout bien tolérées par le patient.

La demanderesse a montré que la composition selon l'invention permet de répondre parfaitement à ce besoin.

La composition selon l'invention est destinée à être administrée directement dans la bouche. L'administration topique de la composition peut être accomplie par application d'une solution, d'une suspension, d'un gel, d'une lotion, d'un lait d'une crème. L'un des moyens possibles et préféré est l'administration de la composition par un spray. D'une manière préférée un spray large facilitant l'application de la composition pour tapisser l'ensemble de la cavité buccale sera choisi.

La fréquence d'utilisation d'un tel spray sera fonction de la composition, de la dose à administrer sur la journée, des besoins du patient, cette fréquence sera déterminée par le médecin et/ou le pharmacien, toutefois une fréquence maximale de trois pulvérisations huit fois par jour est recommandée.

La présente invention concerne donc une composition pharmaceutique telle que décrite dans les revendications pour son utilisation dans le traitement de l'hyposialie ou de l'asialie.

Un objet de la présente invention apparaît donc comme un dispositif médical qui selon l'article L.5211-1 du Code de la Santé Publique se définit comme tout instrument, appareil, équipement, matière ou autre article, destiné à être utilisé chez l'homme à des fins de diagnostic, de prévention, de contrôle, de traitement ou d'atténuation d'une pathologie, et dont l'action principale dans ou sur le corps humain n'est pas obtenue par des moyens pharmacologiques ou immunologiques ni par le métabolisme, mais dont la fonction peut être assistée par de tels moyens.

Par agent humectant on entend au sens de la présente invention, une petite molécule capable d'attirer et/ou de retenir l'eau à proximité ou à disposition des cellules grâce à ses propriétés hygroscopiques.

L'agent humectant peut être avantageusement choisi parmi l'acide pidolique et ses dérivés, le butylène glycol, le gluconate de calcium, le fructose, le glucose, l'isomalt, le lactose, le maltitol, le mannitol, le polydextrose, le sorbitol, le saccharose, le xylitol, le glycérol, l'acide glycyrrhizique et ses dérivés, l'histidine, l'acide hyaluronique et ses sels, les hydrolysats de soie, de kératine ou de soja, les PEG (-7, -8, -10, -12, -14...), le phytantriol, le propylène glycol, la soie, l'urée. De manière préférée, l'agent humectant est le glycérol.

Par agent gélifiant on entend au sens de la présente invention, une substance qui permet de transformer une composition plus ou moins fluide en une composition plus ou moins visqueuse.

L'agent gélifiant peut être avantageusement choisi parmi les polysaccharides d'origine végétale, comme la gomme xanthane et ses dérivés, les copolymères d'alkylacrylates et d'acides acrylique et méthacrylique, notamment obtenus par voie de synthèse, et les silicates, notamment les silicates d'origine minérale plus communément connus sous le nom de Veegum® ou Bentones®. De manière préférée, l'agent gélifiant est la gomme xanthane.

Ainsi selon un mode préféré de réalisation de l'invention, la composition pharmaceutique comprend du glycérol, de la gomme xanthane et un agent filmogène.

Par agent filmogène on entend au sens de la présente invention, une substance qui permet de produire un film continu sur une muqueuse.

L'agent filmogène comprend les filmogènes hydrophobes, comme les huiles minérales, les silicones et les cires synthétiques ou naturelles, on citera comme exemple à titre illustratif l'huile de vaseline ; mais aussi les filmogènes hydrophiles, comme les protéines (collagènes, élastine...), les glucides complexes comme des glucosaminoglycanes (l'acide hyaluronique, la chondroïtine sulfate), la chitosane, des polymères hydrophiles d'origine végétale (galactomannanes, xyloglucanes, polysaccharides, mucopolysaccharides...), des polymères de synthèse, des dérivés des alcools polyvinyliques, le carbopol... On citera comme exemple la polyvinylpyrrolidone avec notamment les différents Kollidon®.

La composition comprend deux agents filmogènes, notamment des dérivés des alcools polyvinyliques tels que spécifiés dans les revendications.

D'une manière encore préférée, l'agent filmogène de la composition est un mélange de polyvinylpyrrolidone et de copolyvidone.

Selon un mode de réalisation de l'invention, la composition comprend au moins un agent humectant, un agent gélifiant et un agent filmogène dans un véhicule pharmaceutiquement acceptable.

Dans la présente invention, on entend désigner par « pharmaceutiquement acceptable » ou « acceptable sur le plan pharmaceutique » ce qui est utile dans la préparation d'une composition pharmaceutique qui est généralement sûr, non toxique et ni biologiquement ni autrement non souhaitable et qui est acceptable pour une utilisation vétérinaire de même que pharmaceutiquement humaine.

Dans un mode de réalisation de l'invention, la composition pharmaceutique comprend en outre au moins un agent solubilisant, ces agents sont bien connus de l'homme du métier. Un agent solubilisant particulièrement adapté à la composition pharmaceutique selon l'invention est l'hydroxystéarate de macrogolglycérol 40.

Dans un mode de réalisation de l'invention, la composition pharmaceutique comprend en outre au moins un mucilage. D'une façon préférée, le mucilage provient d'un extrait de racine de guimauve. On entend par mucilage, une substance végétale constituée de polysaccharides de nature pectique (acide polygalacturonique) qui gonfle au contact de l'eau et qui possède une consistance visqueuse. Les racines primaires de la plupart des plantes sécrètent des mucilages. Les racines de la guimauve sont particulièrement riches en mucilage. Ces derniers sont particulièrement adaptés pour être incorporés dans une composition selon l'invention.

Dans un mode de réalisation de l'invention, la composition pharmaceutique comprend en outre au moins une substance auxiliaire choisie parmi les édulcorants, les antioxydants, les colorants, les arômes.

Un ou plusieurs édulcorants peuvent être choisis parmi le groupe constitué par : l'acésulfame, l'aspartame, l'acide cyclamique et ses sels, l'isomalt, la saccharine et ses sels, le sucralose, l'alitame, la thaumatine, l'acide glycyrrhizique et ses sels, la néohespéridine dihydrochalcone, les glucosides de stéviol, le néotame, le sel d'aspartame-acésulfame, le tagatose, la brazzéine, la curculine, l'hydrolysat d'amidon hydrogéné, la mabinline, la miraculine, le monelline, la pentadine, le sirop de polyglycitol, le maltitol, le sirop de maltitol, le lactitol, le xylitol, l'érythritol, le tréhalose.

Un ou plusieurs antioxydants peuvent être choisis parmi le groupe constitué par : l'acide ascorbique, l'ascorbate de sodium, l'ascorbate de calcium, le diacétate d'ascorbyle, le palmitate d'ascorbyle, le stéarate d'ascorbyle, l'alpha tocophérol, le bêta tocophérol, le gamma tocophérol, le delta tocophérol, les gallates, les composés libérant du dioxyde de soufre comme le métabisulfite de sodium, la résine de gaïac, l'acide érythorbique, l'érythorbate de sodium, l'isoascorbate de potassium ou de calcium, la butylhydroquinone, le butylhydroxyanisole, le butylhydroxytoluène, les dérivés de l'acide éthylènediaminetétracétique et leurs combinaisons.

Les colorants et les arômes peuvent être naturels et/ou artificiels, ils sont bien connus de l'homme du métier.

Dans un mode de réalisation de l'invention, la composition pharmaceutique peut comprendre en outre des agents antimicrobiens tels que des conservateurs ou agents antifongiques choisis parmi le 2-hydroxybiphényl, l'urée de l'imidazolidinyle, l'urée du diazolidinyle, l'hydroxyméthylglycinate de sodium, des dérivés halogénés tels que butylcarbamate d'iodopropynyle, le 2-bromo-2-nitropropan-1,3-diol, 2,4,4'-trichloro-2'-hydroxydiphényléther (triclosan), le 3,4,4'-trichlorocarbanilide (triclocarban), le chlorbutanulum, l'alcool 2,4-dichlorobenzylique, l'urée du N-4-chlorphényl-N'-3,4-dichlorphényle, le 1,2-dibromo-2,4-dicyanobutane, le chloroxylénol, le cétoconazole, l'oxiconazole, le butoconazole, le clotrimazole, l'éconazole, l'énilconazole, le fenticonazole, le miconazole, le sulconazole, le tioconazole, le fluconazole, l'itraconazole, le terconazole, des actifs contenant un ou plusieurs azotes cationiques tels que le chlorure de cétyltriméthylammonium, le chlorure de cétylpyridinium, le chlorure de benzéthonium, le chlorure de diisobutyléthoxyéthyl-diméthylbenzylammonium, le chlorure de diisobutyl-phénoxy-éthoxyéthyl-diméthylbenzylammonium, le chlorure, bromure saccharinate de N-alkyl-N,N-diméthylbenzylammonium, le chlorure de trimétylammonium, l'aluminiumchlorohydroxylacétate de sodium, le chlorure de tricétylméthylammonium, diaminoalkylamide, des agents antimicrobiens insaturés tels que le farnesol, la terbinafine ou la naftifine, des agents aromatiques hétérocycliques tels que le bifonazole, le cloconazole, l'isoconazole, des composés de la famille des parabènes, notamment le méthylparabène, l'éthylparabène, le propylparabène, l'isopropylparabène, le butylparabène, l'isobutylparabène, le benzylparabène, de tout autre agent antimicrobien ou antifongique connu de l'homme du métier et leurs mélanges.

Dans un mode de réalisation de l'invention, la composition pharmaceutique peut également comprendre des composés permettant d'ajuster la zone de pH de ladite composition en évitant des pH acides favorables à la déminéralisation des tissus durs, on parle d'ajusteurs de pH. Cela comprend des agents neutralisants ou bien des agents tampons. Les sels d'acide couramment utilisés sont le citrate de sodium, le phosphate de sodium, le lactate de potassium, le phosphate de potassium ou encore le malate de potassium. On entend par phosphate de sodium l'ensemble des sels de sodium et de phosphate, le dihydrogénophosphate de sodium ou phosphate monosodique, l'hydrogénophosphate de sodium ou phosphate disodique, le phosphate de sodium ou phosphate trisodique. On entend par phosphate de potassium, les sels formés des ions phosphate et potassium résultant de l'attaque de l'hydroxyde de potassium par l'acide phosphorique, le dihydrogénophosphate de potassium monopotassique, l'hydrogénophosphate de potassium dipotassique, le phosphate de potassium, le phosphate dipotassique et tripotassique.

Les compositions peuvent en outre comprendre un ou plusieurs autres ingrédients tels que des tampons de pH, des vitamines, des fragrances, et tout autre composé utile bien connu de l'homme du métier.

Dans un mode de réalisation de l'invention, la composition pharmaceutique peut également comprendre un ou plusieurs agents minéralisants, on peut citer par exemple le chlorure de potassium, le chlorure de sodium, le chlorure de magnésium, le chlorure de calcium, ces agents sont bien connus de l'homme du métier.

Selon l'invention, la quantité de l'agent humectant est comprise de 15 % à 65 % du poids total de la composition pharmaceutique. D'une manière préférée, la quantité de l'agent humectant est comprise de 20 % à 50 % du poids total de la composition pharmaceutique. D'une manière encore préférée, la quantité de l'agent humectant est comprise de 30 % à 40 % du poids total de la composition pharmaceutique. D'une autre manière préférée, la quantité de l'agent humectant est égale à 35 % du poids total de la composition pharmaceutique.

Dans un mode particulier de l'invention, la quantité de l'agent gélifiant est comprise de 0,05 % à 1 % du poids total de la composition pharmaceutique. D'une manière préférée, la quantité de l'agent gélifiant est comprise de 0,1 % à 0,5 % du poids total de la composition pharmaceutique. D'une autre manière préférée, la quantité de l'agent gélifiant est égale à 0,2 % du poids total de la composition pharmaceutique.

Dans un mode particulier de l'invention, la quantité de l'agent filmogène est comprise de 0,1 % à 10 % du poids total de la composition pharmaceutique. D'une manière préférée, la quantité de l'agent filmogène est comprise de 0,1 % à 5 % du poids total de la composition pharmaceutique. D'une autre manière préférée, la quantité de l'agent filmogène est comprise de 0,5 % à 3 % du poids total de la composition pharmaceutique. D'une autre manière préférée, la quantité de l'agent filmogène est égale à 2 % du poids total de la composition pharmaceutique.

Selon un mode préféré de l'invention, la composition pharmaceutique est administrable sous forme de spray. Un spray large est particulièrement adapté pour que l'application de la composition tapisse l'ensemble de la cavité buccale.

Selon un autre mode préféré de l'invention, la composition pharmaceutique est sous forme de présentation pharmaceutique de type dose unique ou encore appelée unidose ou monodose. On peut citer comme exemples les sachets pouvant contenir la composition pharmaceutique selon l'invention sous forme de poudre ou de granules ou même de solution, de suspension ou de gel ; les ampoules en verre à deux pointes ou à une pointe pouvant contenir la composition pharmaceutique selon l'invention sous forme liquide ; des tubes en matière plastique pouvant contenir la composition pharmaceutique selon l'invention sous forme liquide ou sous forme de gel. Ces présentations pharmaceutiques monodoses sont bien connues de l'homme du métier.

Les exemples suivants illustrent l'invention sans en limiter la portée.

### Exemple 1 : caractérisation tribologique de produits salivaires

Le but de cette étude est de caractériser l'effet lubrifiant de différents produits salivaires sur des langues de porc.

### Matériel et méthodes

L'ensemble des tests est réalisé en température et humidité contrôlées dans une enceinte spécifiquement adaptée pour l'étude.

### - Préparation des échantillons de langues

Les langues sont choisies dans un abattoir, elles sont conditionnées, c'est à dire que les zones non utilisées sont détruites. Elles sont ensuite nettoyées avec un sérum physiologique neutre. Elles sont alors découpées pour produire des échantillons homogènes et standards. Les échantillons sont de nouveau nettoyés avec du sérum physiologique neutre, ils sont placés en emballage individuel et congelés.

### - Mesure de la friction

La friction est mesurée à l'aide d'un bio-tribomètre (Zahouani et al., Skin Research and Technology, 15 :68-76, 2009), cet appareil a été spécifiquement conçu pour la réalisation d'expérimentations sur tissus biologiques. Cette technologie permet la mesure, en phase de frottement stabilisé, des efforts normal et tangentiel. Par rapport entre ces deux quantités, le coefficient de frottement τ est calculé. Trois mesures sont réalisées pour chaque substitut salivaire avant et après lubrification. Les effets des produits avant et après lubrification sont analysés d'une part vis à vis de la significativité de l'effet lubrifiant et d'autre part au regard de leur effet lubrifiant en comparaison de celui apporté par la salive humaine.

Dans ce test, 17 compositions différentes ont été testées dont 4 servent de références (tableau 1), la salive humaine, le substitut Aequasyal®, le substitut Biotène® et le substitut Oasis®. Une composition (composition n°1) a été évaluée deux fois pour tester la reproductibilité du test.

**Tableau : compositions testées**

| N° composition | Description |
|---|---|
| Composition 1 | Extrait de lin + 15% glycérol + 0,2% xanthane + cremophor 40 |
| Composition 2 | Extrait de yam + 5% glycérol |
| Composition 3 | Extrait de lin + 30% glycérol + 0,2% xanthane + cremophor 40 |
| Composition 4 | 35% glycérol + 0,2% xanthane + cremophor 60 + copolyvidone |
| Composition 5 | Extrait de lin + 5% glycérol |
| Composition 6 | Mucine bovine + 5% glycérol |
| Composition 7 | Référence commerciale Aequasyal® |
| Composition 8 | Référence commerciale Biotène® |
| Composition 9 | Mucine bovine + 63% glycérol + blanose + carraghenate |
| Composition 10 | Référence commerciale Oasis® |
| Composition 11 | Lécithine + 5% glycérol + 0,2% xanthane |
| Composition 12 | 35% glycérol |
| Composition 13 | 85% silicone + 15% huile de paraffine |
| Composition 14 | Lécithine + 15% glycérol + 0,4% xanthane |
| Composition 15 | Lécithine + 5% glycérol + 0,4% xanthane |
| Composition 16 | 95% silicone + 5% huile de paraffine |
| Composition 17 | Salive humaine |

| | |
|---|---|
| Aequasyal® : triesters de glycérol oxydés, dioxyde de silicium ; Biotène® : glycérol, xylitol, PEG 60, VP/VA copolymère, huile de ricin, xanthane ; Oasis® : glycérol, sorbitol, poloxamère 338, PEG 60, huile de ricin, xanthane. | |

### Résultats

Les mesures basales réalisées avant lubrification révèlent une certaine variabilité. Ces mesures étant faites sur des substrats différents, cette variabilité s'explique facilement. Ainsi la moyenne globale des coefficients de frottement mesurés est de 1,12 assortie d'un écart type de 0,18. La valeur maximale est de 1,51 et la valeur minimale est de 0,73. La dispersion des résultats est non négligeable ce qui induit par conséquent la nécessité de raisonner vis à vis des valeurs absolues des coefficients de frottement et non vis à vis de leurs variations exprimées en pourcentages.

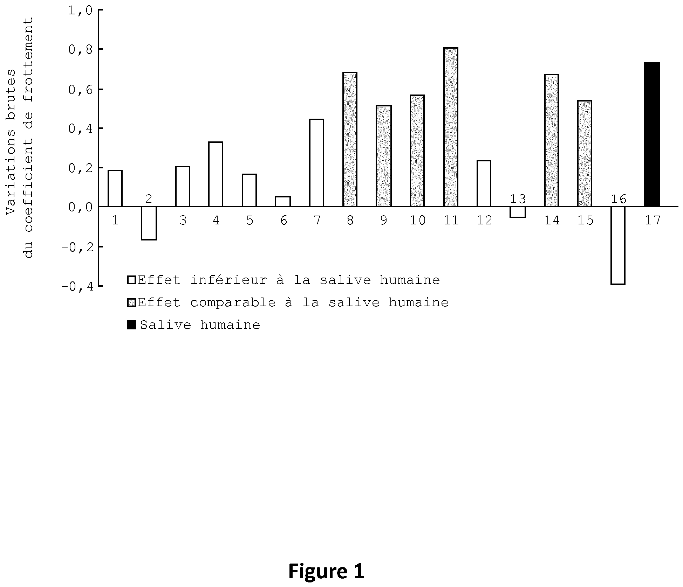

La figure 1 représente l'effet lubrifiant des différentes compositions testées, à savoir la variation du coefficient de frottement obtenu après et avant lubrification par une composition donnée. La composition 17 (salive humaine sert évidemment de référence, colonne noire). Il ressort de cette analyse que 2 groupes peuvent être distingués. Dix compositions (colonnes blanches de la figure 1) présentent un effet significativement inférieur à la salive humaine, dont 3 compositions (2, 13 et 16) se révèlent anti-lubrifiantes. Six compositions (colonnes grisées) présentent un effet lubrifiant comparable à la salive humaine. Parmi ces compositions efficaces on trouve 2 références commerciales, ce qui valide la fiabilité du test. Toutefois une troisième référence commerciale présente un effet lubrifiant moins important, il se révèle même significativement inférieur à la salive humaine, ce test est donc assez restrictif. Il apparaît intéressant de corréler les effets lubrifiants avec les composants de chaque composition salivaire. Les trois substituts salivaires de référence 1, 3 et 5 qui présentent une similarité forte de composition en extrait de lin, glycérol et de gomme xanthane et de crémophor 40 pour les références 1 et 3, ne semblent pas convaincants en terme d'effet lubrifiant. L'absence d'extrait de lin et l'ajout de copolyvidone dans la référence 4 par rapport à la référence 3, tend à améliorer le pouvoir lubrifiant. Cette comparaison permet d'écarter l'extrait de lin comme composant fondamental des substituts. En observant les compositions et résultats associés aux références 13 et 16, il est possible de conclure que l'utilisation d'une base de silicone ne semble pas opportune pour cette problématique. Ces deux produits présentent un effet inverse à celui escompté et proportionnel à la quantité de silicone utilisé pour la formulation. La rhéologie de ce constituant viscoélastique et ses propriétés physico-chimiques, tension de surface par rapport aux capacités adhésives peuvent être mises en cause. De même, l'ajout d'extrait de yam dans la composition de la référence 2 semble induire un effet négatif sur le pouvoir lubrifiant. En observant la composition de la référence 6, il semblerait que la mucine bovine n'exerce pas d'influence sur le pouvoir lubrifiant. Toutefois la référence 9 arrive à mimer de manière satisfaisante le fonctionnement de la salive humaine. La blanose et le carraghenate, tous les deux jouant un rôle d'épaississants participent probablement aux bonnes performances de ce produit. A moins que le fort taux en glycérol soit responsable de cette bonne activité ou performance. Les résultats de la référence 12 semblent montrer l'inverse, mais le glycérol seul ne permet certainement pas d'atteindre un effet suffisant. La comparaison des deux substituts 14 et 15 est également en faveur d'un taux élevé en glycérol pour une bonne activité. Si ces deux références présentent un pouvoir lubrifiant comparable à celui de la salive humaine, le meilleur résultat est trouvé pour la référence 14, contenant 15% de glycérol versus 5% dans la composition 15. La proportion en gomme xanthane peut également être analysée en comparant les références 11 et 15, où la seule différence est la teneur en gomme xanthane, 0,4% versus 0,2%. La référence présentant le meilleur pouvoir lubrifiant il semblerait qu'un taux de 0,2% de gomme xanthane soit suffisant. Finalement seulement quatre compositions (références 9, 11, 14 et 15) arrivent à mimer de manière satisfaisante le fonctionnement de la salive humaine. Si la lécithine est présente dans trois de ces compositions, le glycérol est présent dans toutes ces compositions performantes. Il semble donc tentant de bâtir une composition sur ce constituant fondamental.

Les références commerciales sont représentées par les produits de référence 7, 8 et 10. Ces trois compositions contiennent du glycérol. Mais parmi ces trois substituts, seuls les produits 8 et 10 apparaissent être des substituts satisfaisants. Il est intéressant de noter que dans la référence commerciale Aequasyal® qui se révèle être significativement moins active que la salive humaine, le glycérol se trouve sous forme de triesters de glycérol oxydés. Lors de ce test, une composition a été testée 2 fois pour vérifier la reproductibilité du test. C'est le cas de la référence 1. Les variations brutes (différence du coefficient de frottement avant et après lubrification) sont 0,19 et 0,16, ce qui démontre une bonne reproductibilité de ce test.

### Conclusions

Il ressort de ce test que les références 9, 11, 14 et 15 présentent un pouvoir lubrifiant proche de celui de la salive humaine. Le composant commun à toutes ces compositions semble être le glycérol, un taux élevé de glycérol pourrait même se révéler fondamental.

### Exemple 2 : test sensoriel

Le but de cette étude est d'évaluer la caractéristique sensorielle « sensation de bouche sèche » lors de l'utilisation d'un spray contenant une composition selon l'invention par rapport à un spray concurrent du marché.

Une enquête auprès d'une soixantaine de personnes faisant partie d'un panel d'analyse sensorielle, a été menée afin de dépister des sujets sains présentant une sensation de bouche sèche. Un questionnaire, inspiré de critères européens pour dépister le syndrome de Gougerot-Sjögren a été utilisé afin de dépister des sujets présentant une xérostomie subjective. Ainsi 6 sujets ont été identifiés et ont participé au test sensoriel. La sensation de bouche sèche a été évaluée sur l'échelle visuelle analogique de 0 à 100. Au cours de deux séances successives, les sujets testent selon un plan de randomisation les deux sprays (une seule utilisation par séance). Les sprays sont présentés de manière anonyme, les sujets effectuent soit quatre pulvérisations pour le spray renfermant la composition selon l'invention, correspondant au total à 0,52g de produit administré, soit deux pulvérisations pour le spray Aequasyal® correspondant au total à 0,20g de produit administré.
- Le comparateur est le spray Aequasyal®, dans un conditionnement commercial anonymisé.
- La composition selon l'invention a été pulvérisée à l'aide d'un spray adapté à la composition, décrite ci-dessous :

| Ingrédients | Quantité (%) |
|---|---|
| Glycérol | 35 |
| Kollidon VA64 | 0,5 |
| Hydroxystéarate de macrogolglycérol 40 | 1,0 |
| Gomme xanthane | 0,2 |
| Xylitol | 0,5 |
| Alcool benzylique | 0,8 |
| Sorbate de potassium | 0,4 |
| Arôme | 0,02 |
| Acide lactique dilué ¼ | qs pH 6,25 |
| Eau | qs 100 |

Un questionnaire informatique présente les échelles de mesures analogiques aux différentes phases de l'expérience. L'étude consiste en 6 phases successives, la phase 1 est la sensation de bouche sèche avant la pulvérisation, la phase 2, immédiatement après la pulvérisation, la phase 3 est la sensation de bouche sèche 5 minutes après la pulvérisation, la phase 4, 10 minutes après la pulvérisation, la phase 5, 15 minutes après, la phase 6, 20 minutes après la pulvérisation, la phase 7, 25 minutes après, la phase 8, 30 minutes après, la phase 9, 35 minutes après et la phase 10, 40 minutes après la pulvérisation. Chaque sujet teste le produit imposé par l'ordre de randomisation, il déplace un curseur sur une échelle de 0 à 100 en fonction de l'intensité ressentie à chacune des phases.

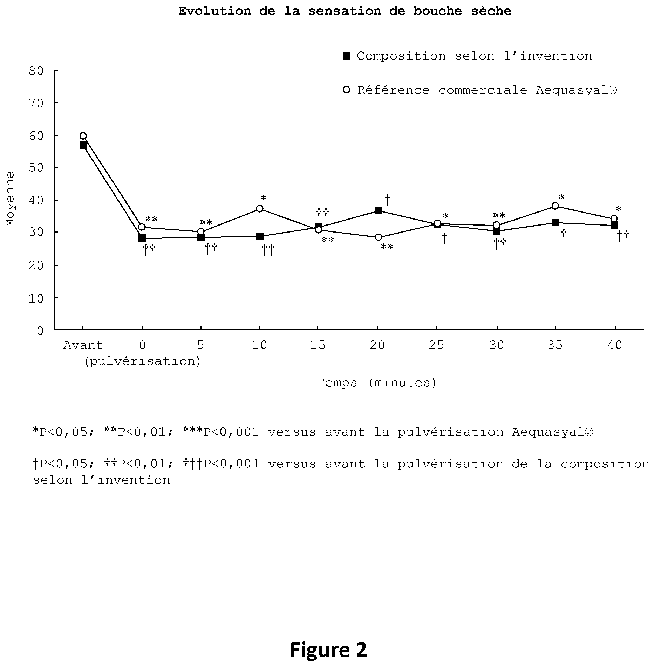

Les résultats de cette étude sensorielle sont résumés sur la figure 2. En analyse temps par temps, la composition selon l'invention administrée par spray permet de diminuer significativement la sensation de bouche sèche pour tous les temps étudiés, 40 minutes après les quatre pulvérisations la sensation de bouche sèche est toujours significativement atténuée. La composition commerciale Aequasyal® diminue sensiblement de la même manière la sensation de bouche sèche que la composition selon l'invention.

### Exemple 3 : composition selon l'invention

A titre d'illustration de l'objet de la présente invention, on mentionnera un autre exemple de composition.

| Ingrédients | Quantité (%) |
|---|---|
| Hydroxystéarate de macrogolglycérol 40 | 1,00 |
| Glycérol | 35,00 |
| Gomme xanthane | 0,20 |
| Xylitol | 0,25 |
| Kollidon VA64 | 0,50 |
| Kollidon 30 | 1,50 |
| Guimauve, hydroglycérinée | 1,00 |
| Sorbate de potassium | 0,40 |
| Alcool benzylique | 0,80 |
| Chlorure de potassium | 0,12 |
| Phosphate disodique | 0,56 |
| Phosphate de potassium | 0,54 |
| Eau | 58,13 |

### Exemple 4 : étude clinique

Le but est d'évaluer l'efficacité clinique et la sécurité d'une composition selon l'invention dans le soulagement de la xérostomie chez des patients présentant une hyposialie iatrogène, une référence commerciale Aequasyal® a également été testée en guise de comparaison.

### Méthodologie

L'étude a été conduite chez des patients présentant une xérostomie induite par des médicaments, l'hyposialie iatrogène a été objectivée par un test salivaire classique avant leur inclusion dans l'étude. Cette étude a été faite dans un seul centre, 24 patients ont été inclus, l'efficacité qui doit être très rapide est testée sur une seule journée. Deux compositions sont évaluées :
- composition selon l'invention, composition de l'exemple 3,
- référence commerciale, Aequasyal®.

Une demi-heure après un petit déjeuner, le produit est administré à l'aide d'un spray, 3 pulvérisations pour la composition selon l'invention, ce qui correspond à 0,6 g de produit administré, et 2 pulvérisations pour la référence commerciale, correspondant à 0,2 g de produit administré.

Une seconde administration des produits est réalisée 4 heures après (3 ou 2 pulvérisations selon les compositions) une demi-heure avant le repas de midi. La sensation de bouche sèche est évaluée par un questionnaire informatique qui présente des échelles de mesures analogiques (même évaluation que pour l'exemple 2) allant de 0 à 100, 0 étant pas de sensation de bouche sèche et 100 étant la pire sensation imaginable de bouche sèche. L'évaluation se fait sur au moins 6 heures. Après cette journée de test, une période de « wash out » de 1 à 3 jours est laissée aux patients qui reviennent ensuite pour une seconde évaluation, les patients qui ont testé la composition selon l'invention testent alors la référence commerciale et vice versa. Pendant la durée du test, d'autres critères ont été évalués, comme par exemple la difficulté à parler, la difficulté à avaler, mais aussi l'acceptabilité de la composition, les effets secondaires potentiels ont également été évalués.

### Résultats

### - Sensation de sécheresse de la bouche

Avant la première administration du produit, tous les sujets se plaignaient d'une sécheresse buccale modérée, c'est-à-dire avec une réponse égale ou supérieure à 40 mm sur l'échelle de mesure analogique allant de 0 à 100 mm. L'hyposalivation avait été confirmée par une mesure du débit salivaire (dans des conditions de non stimulation) et qui devait être inférieur ou égal à 0,5 mL/min.

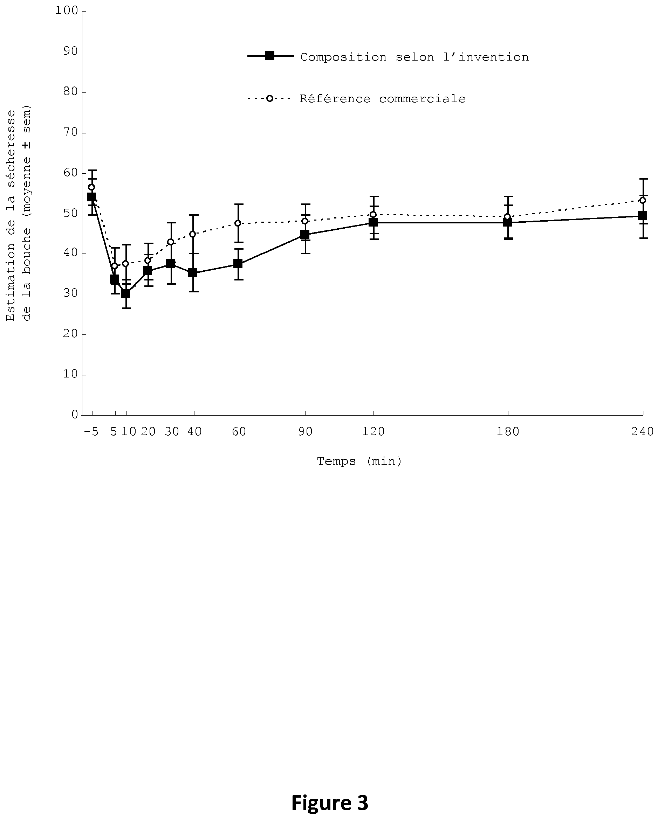

Les résultats illustrés dans la figure 3 montrent une réduction similaire sur cette échelle de mesure entre la composition selon l'invention et la référence commerciale. Une diminution rapide de la sensation de sécheresse buccale est observée dès les cinq premières minutes après l'application des composés, avec une réponse légèrement meilleure avec la composition selon l'invention et ce jusqu'à 90 minutes. L'effet de la seconde application faite avant le repas avait pour but de faciliter la prise du repas et notamment la facilité pour avaler. Le niveau de réduction de la sécheresse buccale est substantiel pour les deux produits évalués. Le tableau ci-dessous montre la durée d'action de l'effet qui est défini comme une réduction de 20% de la sensation de sécheresse buccale après la première et la seconde application.

| Sécheresse buccale | | Composition selon l'invention | Référence commerciale |
|---|---|---|---|
| Première administration | Moyenne | 1 h 29 min | 1 h 08 min |
| Seconde administration | Moyenne | 33 min | 33 min |

La composition selon l'invention se révèle avoir une durée d'action plus longue que celle de la référence commerciale. Après le repas, cette durée d'action est raccourcie pour les deux formulations, probablement à l'ingestion plus rapide des produits au cours du repas.

### - Autres critères d'évaluation

D'autres symptômes comme la difficulté à parler, la difficulté à avaler, la sécheresse de la gorge et la sécheresse des lèvres sont également évalués. La composition selon l'invention présente un profil d'efficacité très intéressant.

### - Tolérance

Aucun effet secondaire majeur n'a été reporté pendant l'étude. Des évènements mineurs notamment au niveau digestif ont été reportés (un dans chaque groupe), la composition selon l'invention est donc très bien tolérée.

### - Acceptabilité

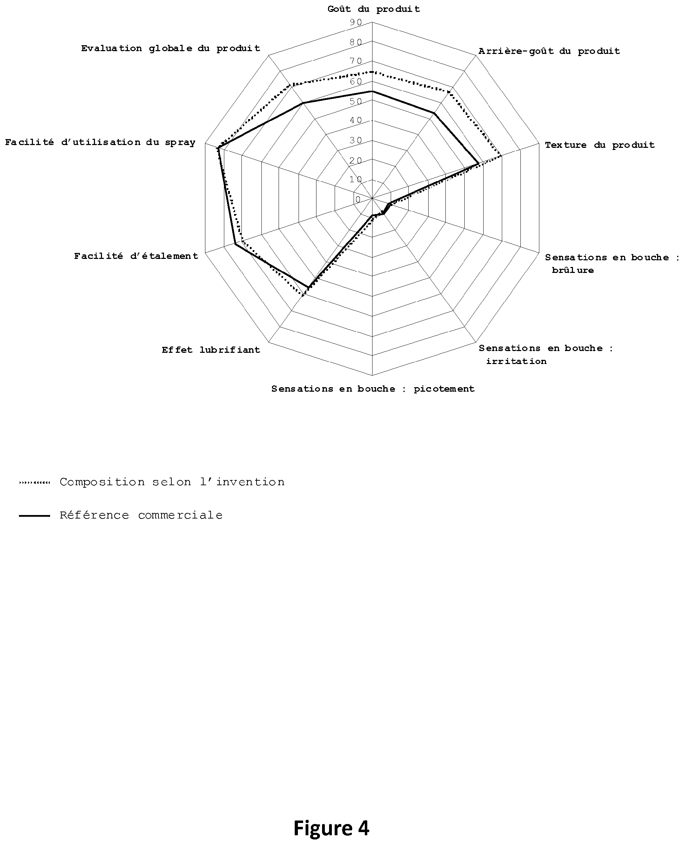

La figure 4 résume l'estimation faite par les patients des deux produits testés avec différents items comme le goût du produit son arrière-goût, sa texture ou l'évaluation globale du produit. La composition selon l'invention est jugée favorablement pour l'ensemble des critères examinés. Très peu de mauvaises sensations en bouche comme les picotements, les irritations sont ainsi reportées. La composition selon l'invention est même mieux notée que la référence commerciale pour plusieurs critères très importants, comme le goût du produit, son arrière goût, sa texture, son effet lubrifiant et même sur l'évaluation globale du produit. Elle apparait légèrement en retrait uniquement sur un critère qui est la facilité d'étalement du produit.

En conclusion, cette étude clinique démontre clairement que la composition selon l'invention possède une réelle efficacité en diminuant la sensation de bouche sèche de façon au moins égale à la référence commerciale et présente même une durée d'action plus longue. Elle est très bien tolérée et les patients la juge de façon très positive sur le plan de l'acceptabilité.

## Revendications

1. Composition pharmaceutique comprenant au moins un agent humectant à raison de 15 % à 65 % en poids du poids total de la composition, un agent gélifiant et un mélange de polyvinylpyrrolidone et de copolyvidone à titre d'agent filmogène, pour son utilisation dans le traitement de l'hyposialie ou de l'asialie.

2. Composition pharmaceutique pour l'utilisation selon la revendication 1 **caractérisée en ce que** l'agent humectant est le glycérol.

3. Composition pharmaceutique pour l'utilisation selon l'une quelconque des revendications 1 ou 2, **caractérisée en ce que** l'agent gélifiant est la gomme xanthane.

4. Composition pharmaceutique pour l'utilisation selon l'une quelconque des revendications 1 à 3, **caractérisée en ce que** la quantité de l'agent gélifiant est comprise de 0,05 % à 1 %, de préférence de 0,1 % à 0,5 % du poids total de la composition.

5. Composition pharmaceutique pour l'utilisation selon l'une quelconque des revendications 1 à 4, **caractérisée en ce que** la quantité d'agent filmogène est comprise de 0,1 % à 5 %, de préférence de 0,5 % à 3% du poids total de la composition.

6. Composition pharmaceutique pour l'utilisation selon l'une quelconque des revendications 1 à 5, **caractérisée en ce qu'**elle comprend en outre un mucilage.

7. Composition pharmaceutique pour l'utilisation selon la revendication 6, **caractérisée en ce que** le mucilage provient d'un extrait de racine de guimauve.

8. Composition pharmaceutique pour l'utilisation selon l'une quelconque des revendications 1 à 7, **caractérisée en ce qu'**elle comprend en outre un ou plusieurs ajusteurs de pH.

9. Composition pharmaceutique pour l'utilisation selon l'une quelconque des revendications 1 à 8, **caractérisée en ce qu'**elle comprend en outre au moins un agent minéralisant.

10. Composition pharmaceutique pour l'utilisation selon l'une quelconque des revendications 1 à 9, **caractérisée en ce qu'**elle est présentée sous une forme administrable sous forme de spray.

11. Composition pharmaceutique pour l'utilisation selon l'une quelconque des revendications 1 à 10, **caractérisée en ce qu'**elle est sous forme de présentation pharmaceutique de type monodose.

## Patentansprüche

1. Pharmazeutische Zusammensetzung, umfassend mindestens ein Feuchtmittel im Umfang von 15 bis 65 Gew.-% des Gesamtgewichts der Zusammensetzung, einen Gelbildner und ein Gemisch aus Polyvinylpyrrolidon und aus Copolyvidon als Filmbildner für ihre Verwendung bei der Behandlung der Hyposialivation oder der Asialie.

2. Pharmazeutische Zusammensetzung für die Verwendung nach Anspruch 1, **dadurch gekennzeichnet, dass** das Feuchtmittel das Glycerol ist.

3. Pharmazeutische Zusammensetzung für die Verwendung nach einem der Ansprüche 1 oder 2, **dadurch gekennzeichnet, dass** der Gelbildner das Xanthan ist.

4. Pharmazeutische Zusammensetzung für die Verwendung nach einem der Ansprüche 1 bis 3, **dadurch gekennzeichnet, dass** die Menge des Gelbildners von 0,05% bis 1%, vorzugsweise von 0,1% bis 0,5% des Gesamtgewichts der Zusammensetzung liegt.

5. Pharmazeutische Zusammensetzung für die Verwendung nach einem der Ansprüche 1 bis 4, **dadurch gekennzeichnet, dass** die Filmbildnermenge von 0,1% bis 5%, vorzugsweise von 0,5% bis 3% des Gesamtgewichts der Zusammensetzung liegt.

6. Pharmazeutische Zusammensetzung für die Verwendung nach einem der Ansprüche 1 bis 5, **dadurch gekennzeichnet, dass** sie ferner einen Schleimstoff umfasst.

7. Pharmazeutische Zusammensetzung für die Verwendung nach Anspruch 6, **dadurch gekennzeichnet, dass** der Schleimstoff von einem Eibischwurzelextrakt herrührt.

8. Pharmazeutische Zusammensetzung für die Verwendung nach einem der Ansprüche 1 bis 7, **dadurch gekennzeichnet, dass** sie ferner einen oder mehrere pH-Regulatoren umfasst.

9. Pharmazeutische Zusammensetzung für die Verwendung nach einem der Ansprüche 1 bis 8, **dadurch gekennzeichnet, dass** sie ferner mindestens einen mineralisierenden Stoff umfasst.

10. Pharmazeutische Zusammensetzung für die Verwendung nach einem der Ansprüche 1 bis 9, **dadurch gekennzeichnet, dass** sie in einer verabreichbaren Form in Sprayform vorliegt.

11. Pharmazeutische Zusammensetzung für die Verwendung nach einem der Ansprüche 1 bis 10, **dadurch gekennzeichnet, dass** sie in Form einer pharmazeutischen Aufmachung vom Typ Einzeldosis vorliegt.

## Claims

1. A pharmaceutical composition comprising at least one humectant in an amount from 15% to 65% by weight based on the total weight of the composition, one gelling agent and a mixture of polyvinylpyrrolidone and of copolyvidone as a film-forming agent, for its use in the treatment of hyposialia or asialia

2. The pharmaceutical composition for use according to claim 1, **characterized in that** the humectant is glycerol.

3. The pharmaceutical composition for use according to any one of claims 1 or 2, **characterized in that** the gelling agent is xanthan gum.

4. The pharmaceutical composition for use according to any one of claims 1 to 3, **characterized in that** the amount of the gelling agent is comprised from 0.05% to 1%, preferably from 0.1% to 0.5% of the total weight of the composition.

5. The pharmaceutical composition for use according to any one of claims 1 to 4, **characterized in that** the amount of film-forming agent is comprised from 0.1% to 5%, preferably from 0.5% to 3% of the total weight of the composition.

6. The pharmaceutical composition for use according to any one of claims 1 to 5, **characterized in that** it further comprises a mucilage.

7. The pharmaceutical composition for use according to claim 6, **characterized in that** the mucilage comes from a marshmallow root extract.

8. The pharmaceutical composition for use according to any one of claims 1 to 7, **characterized in that** it further comprises one or several pH adjusters.

9. The pharmaceutical composition for use according to any one of claims 1 to 8, **characterized in that** it further comprises at least a mineralizing agent.

10. The pharmaceutical composition for use according to any one of claims 1 to 9, **characterized in that** it appears in an administrable form as a spray.

11. The pharmaceutical composition for use according to any one of claims 1 to 10, **characterized in that** it is in the form of a pharmaceutical presentation of the monodose type.
